(19) Europäisches Patentamt
European Patent Office
Office européen des brevets

(11) Veröffentlichungsnummer: **0 243 615 B1**

# (12) EUROPÄISCHE PATENTSCHRIFT

(45) Veröffentlichungstag der Patentschrift: **20.03.91**

(51) Int. Cl.5: **C07D 233/90**, A01N 43/50

(21) Anmeldenummer: **87103004.5**

(22) Anmeldetag: **03.03.87**

Die Akte enthält technische Angaben, die nach dem Eingang der Anmeldung eingereicht wurden und die nicht in dieser Patentschrift enthalten sind.

## (54) 1-Phenylimidazolcarbonsäureamide, ihre Herstellung sowie ihre Verwendung als Wachstumsregulatoren.

(30) Priorität: **12.03.86 DE 3608143**

(43) Veröffentlichungstag der Anmeldung:
**04.11.87 Patentblatt 87/45**

(45) Bekanntmachung des Hinweises auf die Patenterteilung:
**20.03.91 Patentblatt 91/12**

(84) Benannte Vertragsstaaten:
**AT CH DE ES GR IT LI NL SE**

(56) Entgegenhaltungen:
**EP-A- 0 137 868**
**EP-A- 0 167 776**
**EP-A- 0 185 961**
**EP-A- 0 199 206**

(73) Patentinhaber: **HOECHST AKTIENGESELLSCHAFT**
**Postfach 80 03 20**
**W-6230 Frankfurt am Main 80(DE)**

(72) Erfinder: **Schmierer, Roland, Dr.**
**An der Weinleite 5a**
**W-8901 Todtenweis(DE)**
Erfinder: **Handte, Reinhard, Dr.**
**Theilweg 23**
**W-8901 Gablingen(DE)**
Erfinder: **Mildenberger, Hilmar, Dr.**
**Fasanenstrasse 24**
**W-6233 Kelkheim (Taunus)(DE)**
Erfinder: **Bürstell, Helmut, Dr.**
**Am Hohlacker 65**
**W-6000 Franfurt am Main(DE)**

**EP 0 243 615 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

1-Phenyl-imidazol-5-carbonsäurederivate sowie deren Verwendung als Fungizide, Herbizide und Pflanzenwachstumsregulatoren sind aus DE-A 32 17 094 bekannt.

Diese Verbindungen besitzen die allgemeine Formel (A)

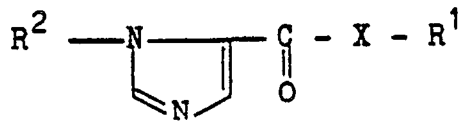

(A)

worin $R^1$ H, Phenyl, Alkyl, Alkenyl, die beide durch Halogen, Alkoxy oder Dialkylamino substituiert sein können, in Metallkation oder Ammonium, $R^2$ ein substituierter Phenylrest und X = O,S oder N bedeuten und im Falle X = N zwei Reste $R^1$ an X gebunden sind.

Es wurde nun überraschenderweise gefunden, daß ausgewählte 1-Phenylimidazolcarbonsäureamide, die in der DE-A 32 17 094 nicht beschrieben sind, besonders intensive wachstumsregulierende Wirkungen insbesondere in Reiskulturen besitzen.

Gegenstand der vorliegenden Erfindung sind daher die Verbindungen der Formel I

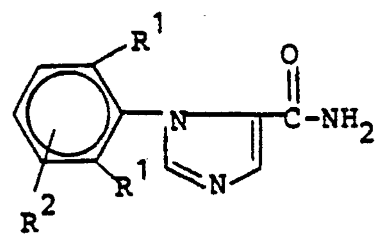

(I)

worin

- $R^1$ unabhängig voneinander $(C_1\text{-}C_3)$Alkyl und
- $R^2$ H, Methyl oder Halogen bedeuten sowie deren in der Landwirtschaft einsetzbaren Salze.
- $R^1$ bedeutet insbesondere Ethyl,
- $R^2$ insbesondere Wasserstoff.

Gegenstand der Erfindung ist ferner ein Verfahren zur Herstellung der Verbindung der Formel I, dadurch gekennzeichnet, daß man eine Verbindung der Formel II,

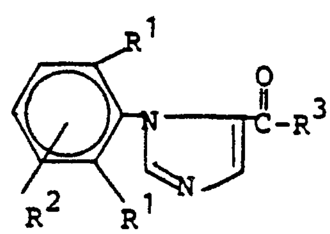

(II)

worin $R^1$, $R^2$ die vorgenannten Bedeutungen besitzen, $R^3$ = einen R est der Formeln

$$-O-R^4,\ -OH,\ Cl,\ -O-\overset{\overset{O}{\|}}{C}-R^4 \text{ oder } -O-\overset{\overset{O}{\|}}{C}-O-R^4$$

und $R^4$ = $(C_1\text{-}C_8)$Alkyl bedeuten, einer Ammonolyse unterwirft und die erhaltenen Verbindungen gewünschtenfalls in ihre Salze überführt.

Die Ammonolysen können nach allgemein üblichen Methoden durchgeführt werden, beispielsweise

- durch Umsetzung der Verbindungen II mit $R^3$ = $OR^4$ mit Ammoniak oder mit Substanzen, die Ammoniak beim Erhitzen freisetzen, wie Harnstoft, Formamid oder $(NH_4)_2CO_3$, gegebenenfalls unter Druck, bei Temperaturen zwischen 100 und 250° C (z.B. Houben-Weyl VIII, S. 656 ff.); oder
- durch Überführen der Verbindungen der Formel II mit $R^3$ = OH in ein Ammoniumsalz, das

anschließend thermisch zersetzt wird. Das Ammoniumsalz kann beispielsweise durch Umsetzen der Säuren mit Ammoniak oder mit den obengenannten Substanzen, die Ammoniak freisetzen, hergestellt werden (z.B. Houben-Weyl VIII, S. 645 ff.); oder

- durch Umsetzung der Verbindungen der Formel II mit $R^3$ =

$$Cl,\ -O-\overset{O}{\overset{\|}{C}}-R^4 \text{ oder } O-\overset{O}{\overset{\|}{C}}-OR^4$$

mit wässrigem oder gasförmigen Ammoniak gegebenenfalls in Anwesenheit eines inerten Lösungsmittels bei 0° bis 100° C (z.B. Organikum, 1973, S. 453 ff.)

Die für die Landwirtschaft verwendbaren Salze der Formel I sind Imidazoliumsalze; diese lassen sich nach bekannten Verfahren mit starken Säuren wie z.B. Salzsäure, Schwefelsäure, Methansulfonsäure, Chlorethanphosphonsäure oder Trichloressigsäure bei einem pH-Wert von $\leq 2$ herstellen.

Mit den erfindungsgemäßen Verbindungen sind typische wachstumsregulierende Effekte erzielbar, die bereits bei niedrigen Dosierungen einsetzen können. Sie greifen regulierend in den pflanzeneigenen Stoffwechsel ein und können damit zur gezielten Beeinflussung von Pflanzeninhaltsstoffen, zur Steigerung des Ertrages, sowie zur Ernteerleichterung und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirkung der Verbindungen als Wuchshemmer in Reis.

Gegenstand der Erfindung sind auch das Pflanzenwachstum regulierende Mittel, die sich durch einen wirksamen Gehalt mindestens einer der erfindungsgemäßen Verbindungen auszeichnen.

Die erfindungsgemäßen Verbindungen können, gegebenenfalls im Gemisch mit weiteren Wirkkomponenten, als Spritzpulver, emulgierbare Konzentrate, versprühbare Lösungen, Stäubemittel, Dispersionen, Granulate oder Mikrogranulate in den üblichen Zubereitungen angewendet werden.

Spritzpulver sind in Wasser gleichmäßig dispergierbare Präparate, die neben dem (den) Wirkstoff(en) außer gegebenenfalls einem Verdünnungs- oder Inertstoff oder Netzmittel wie polyoxethylierte Fettalkohole, Alkyl- oder Alkylphenylsulfonate und/oder Dispergierhilfsmittel wie ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen der Wirkstoffe in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkyl-arylsulfonsaure Calciumsalze wie Ca-dodecylbenzolsulfonat oder nichtionische Emulgatoren wie Fettsäurepolyglykolester, Alkylarylpolyglykolether, Fettalkoholpolyglykolether, PropylenoxidEthylenoxid-Kondensationsprodukte, Fettalkohol-Propylenoxid-Kondensationsprodukte, Alkylpolyglykolether, Sorbitanfettsäureester, Polyoxyethylensorbitansäureester oder Polyoxethylensorbitester.

Stäubemittel kann man durch Vermahlen der Wirkstoffe mit feinverteilten, festen, Stoffen, z.B. Talkum, natürlichen Tonen wie Kaolin, Bentonit, Pyrophillit oder Diatomeenerde erhalten. Granulate können entweder durch Verdüsen der Wirkstoffe auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 5 bis 90 Gew.-%, der Rest zu 100 Gew.-% besteht aus üblichen Formulierungsbestandteilen. Bei emulgierbaren Konzentraten kann die Wirkstoffkonzentration etwa 3 bis 80 Gew.-% betragen. Staubförmige Formulierungen enthalten meistens 0,025 bis 20 Gew.-% an Wirkstoff(en), versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmilltel, Füllstoffe usw. verwendet werden. Daneben enthalten die genannten Wirkstofformulierungen gegebenen falls die jeweils üblichen Haft-, Netz-, Dispergier-, Emulgier-, Penetrations-, Lösemittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Losüngen werden vor der Anwendung üblicherweise nicht mehr mit weiteren inerten Stoffen verdünnt.

Die Aufwandmenge der erfindungsgemäßen Verbindungen variiert im allgemeinen zwischen 0,02 bis 2,5 kg Wirksubstanz pro Hektar, vorzugsweise 0,05 bis 1,5 kg/ha.
Die erfindungsgemäßen Verbindungen, insbesondere die in den Beispielen genannten Verbindungen, lassen sich bei ihrem praktischen Einsatz gegebenenfalls auch vorteilhaft mit bekannten Wachstumsregulatoren kombinieren. Solche bekannten Wachstumsregulatoren sind die Verbindungen der Formel

$R\text{-}CH_2\text{-}CH_2\text{-}N^+(CH_3)_3Cl$

worin R = OH oder Cl bedeutet (common name Chlormequat für R = Cl), ferner N,N-Dimethylpiperidiniumchlorid (Mepiquat, Chlorid, α-Cyclopropyl-4-methoxy-α-(pyrimidin-5-yl)benzylalkohol (Ancymidol), (3aα, 4β, 4aα, 6aα, 7β, 7aα)-1-4-chlorophenyl)-3a, 4, 4a, 6a, 7, 7a-hexahydro-4,7-methano-1H-[1,2]diazeto[3,4-f]-benzotriazol (Tetcylacis), Bernsteinsäure-mono-2,2-dimethylhydrazid (Diaminoazide), 6-Hydroxy-2H-pyridazin-3-on (Maleinsäureanhydrid, 2-Chlor-9-hydroxy-9H-fluoren-9-carbonsauren (Chlorflurenol), 5'-(Trifluormethansulfonamido)acetat-2',4'-xylidid (Mefluidid), 2-Chlorethylphosphonsäure (Ethephon).

Die wachstumsregulatorischen Wirkungen dieser Verbindungen sind beschrieben in Plant Growth Regulator Handbook of the Plant Growth Regulator Working Group 2d. Ed. 1981.

Anstelle der Verbindungen Chlormequat oder Mepiquat-chlorid können prinzipiell auch vergleichbare Salze, die anstelle des Chloridions ein anderes übliches Anion wie Bromid, Nitrat oder Sulfat enthalten, eingesetzt werden.

Kombinationen der Verbindungen I, insbesondere die des Beispiels 1 oder dessen Salze mit Ethephon, lassen sich auch vorteilhaft in den Kulturen des gemäßigten Klimas wie bei Cruziferen (z.B. Raps) oder Gramineen (z.B. Gerste) einsetzen.

Die Kombinationen können sowohl als Mischformulierungen der Komponenten vorliegen, die dann in üblicher Weise mit Wasser verdünnt zur Anwendung gebracht werden; oder sie können als sogenannte Tankmischungen durch gemeinsame Verdünnung der getrennt formulierten Komponenten mit Wasser hergestellt werden; es besteht auch die Möglichkeit, die Komponenten nacheinander zur Anwendung zu bringen, d.h. es werden dann die Komponenten in Einzelformulierungen appliziert.

Die Verbindungen der allgemeinen Formel (I) können auch mit natürlich oder pflanzlichen Hormonen wie Auxinen oder Cytokinen kombiniert werden.

## Formulierungsbeispiele

### Beispiel 1

Ein Stäubemittel wird erhalten, indem man a) 10 GT (GT = Gewichtsteile) Wirkstoff mit 90 GT Talkum oder einem anderen Inertstoff mischt und in einer Schlagmühle zerkleinert, oder indem man b) 60 GT Wirkstoff, 35 GT Talkum und 5 GT Haftmittel (z.B. ein Polysaccharid wie (R)Rhodopol der Rhone-Poulenc S.A.) in der gleichen Weise homogenisiert.

### Beispiel 2

Ein in Wasser leicht dispergierbares, benetzbares Pulver wird erhalten, indem man 25 GT Wirkstoff, 64 GT kaolinhaltigen Quarz als Inertstoff, 10 GT ligninsulfonsaures Kalium und 1 GT oleoylmethyltaurinsaures Natrium als Netz-und Dispergierhilfsmittel mischt und in einer Stiftmühle mahlt. Eine Formulierung mit 5% Wirkstoffgehalt kann wie folgt zusammengesetzt sein: 5% Wirkstoff. 6% eines sulfonierten Naphthalinformaldehydkonensats (z.B. (R)Dispersogen A der Hoechst AG), 2% eines Na-Salzes einer Alkylnaphthalinsulfonsäure (z.B. (R)Leonil DB der Hoechst AG), 5% eines Gemisches aus Polypropylenglykol und $SiO_2$ (z.B. (R)Acrotin 341 der Hoechst AG), 25% eines Silikats (z.B. (R)Sipernat der Degussa AG) und 57% Kaolin Typ 1777.

### Beispiel 3

Ein in Wasser leicht dispergierbares Dispersionskonzentrat wird erhalten, indem man 20 GT Wirkstoff mit 6 GT eines Alkylphenolpolyglykolethers (z.B. [(R)]Triton X 207 von Rohm and Haas Co.), 3 GT Isotridecanolpolyglykolether (8 Ethylenoxid-Einheiten) und 71 GT paraffinischem Mineralöl (Siedebereich ca. 255 bis über 377° C) mischt und in einer Reibkugelmühle auf eine Feinheit von unter 5µm vermahlt.

1) GT = Gewichtsteile

Beispiel 4

Ein emulgierbares Konzentrat wird erhalten aus 15 GT Wirkstoff(e), 75 GT Cyclohexanon als Lösemittel und 10 GT oxethyliertem Nonylphenol (10 Ethylenoxid-Einheiten) als Emulgator.

GT = Gewichtsteile

## Chemische Beispiele

### Beispiel 1

1-(2,6-Diethylphenyl)-imidazol-5-carboxamid

24,4 g (0,1 Mol) 1-(2,6-Diethylphenyl)-imidazol-5-carbonsäure wurden portionsweise zu 36 g (0,3 Mol) Thionylchlorid und 1 ml Dimethylformamid gegeben. Man erhitzte nach der Zugabe bis zur Beendigung der Gasentwicklung auf 80° C, ließ abkühlen, trocknete den festen Rückstand im Vakuum und gab ihn portionsweise unter gutem Rühren in 150 ml eiskalte konzentrierte wässrige Ammoniaklösung. Es wurde 1 Stunde bei Raumtemperatur gerührt; der Niederschlag wurde abfiltriert und getrocknet. Man erhielt 20, 2 g 1-(2,6-Diethylphenyl)-imidazol-5-carboxamid als farblosen Feststoff vom Schmelzpunkt 171 - 171,5° C (Essigester-Hexan); Ausbeute: 83% d. Theorie.

Weitere Beispiele sind in Tabelle 1 aufgeführt.

**Tabelle 1** **Imidazol-5-carboxamide**

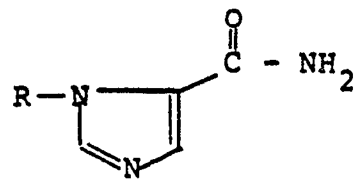

| Beispiel-Nr. | R | Fp (°C) |
|---|---|---|
| 2 | 2,6-Dimethyl-phenyl | |
| 3 | 2-Ethyl-6-methyl-phenyl | 150-154 |
| 4 | 2-Ethyl-6-isopropyl-phenyl | 198-203 |
| 5 | 2-Isopropyl-6-methyl-phenyl | |
| 6 | 2,6-Diisopropyl-phenyl | |
| 7 | 2,6-Diethyl-phenyl (Hydrochlorid) | Harz |
| 8 | " (Chlorethan-phosphonat) | Harz |
| 9 | 2,6-Diethyl-3-methyl-phenyl | 147-151 |
| 10 | 2,6-Diethyl-4-methyl-phenyl | |
| 11 | 2,6-Diethyl-4-brom-phenyl | |
| 12 | 2,6-Diethyl-3-chlor-phenyl | 188-193 |
| 13 | 2,6-Diethyl-phenyl (Hydrogensulfat) | 178 (Zers.) |
| 14 | 2,6-Diethyl-phenyl (Methansulfonat) | 182-186 |
| 15 | 2,6-Diethyl-phenyl (Trichloracetat) | 80 (Zers.) |

Biologische Beispiele

Wuchshemmung in Wasserreis

Reispflanzen wurden angezogen und im Stadium der maximalen Bestockung mit den erfindungsgemäßen Verbindungen sowie mit einer Vergleichsverbindung aus DE-A 32 17 094 behandelt. Die Substanzen wurden sowohl durch Spritzung appliziert als direkt auch in das Wasser gegeben.

Drei Wochen nach Behandlung wurde bei allen Pflanzen der Zuwachs gemessen und die Wuchshemmung in % des Zuwachses der Kontrollpflanzen berechnet. Es wurde außerdem auf eine mögliche phytotoxische Wirkung der Verbindungen geachtet. Die Wuchshemmung wird als prozentualer Wert ermittelt, wobei 100 % den Stillstand des Wachstums und 0 % ein Wachstum entsprechend dem der unbehandelten Kontrollpflanzen bedeuten.

Tabelle 2

| Verbindungen nach Beispiel Nr. | Anwendungs-konz. (kg/ha) | Wuchshemmung % | Phytotox. Wirkung |
|---|---|---|---|
| 1 | 1,25 | 35 | keine |
| | 0,62 | 29 | Schäden |
| 3 | 1,25 | 12 | Schäden |
| | 0,62 | 7 | Schäden |
| 4 | 1,25 | 16 | keine |
| | 0,62 | 11 | Schäden |
| 7 | 1,25 | 32 | keine |
| | 0,62 | 27 | Schäden |
| 8 | 1,25 | 31 | keine |
| | 0,62 | 25 | Schäden |
| 13 | 1,25 | 29 | keine |
| | 0,62 | 24 | Schäden |
| 14 | 1,25 | 26 | keine |
| | 0,62 | 21 | Schäden |
| 15 | 1,25 | 27 | keine |
| | 0,62 | 20 | Schäden |
| A | 1,25 | 11 | keine |
| | 0,62 | 7 | Schäden |

**Ansprüche**

1. Verbindungen der Formel I

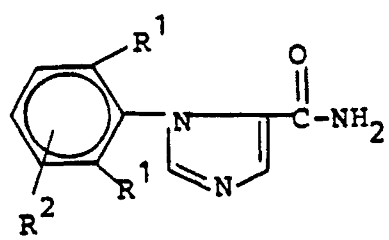

(I)

worin
$R^1$ = unabhängig voneinander $(C_1\text{-}C_3)$Alkyl,
$R^2$ = H, Methyl oder Halogen bedeuten
sowie deren für landwirtschaftliche Zwecke einsetzbaren Salze.

2. Verbindungen der Formel I oder deren Salze, worin

$R^2$ = H bedeutet.

3. Verfahren zur Herstellung von Verbindungen der Formel I und deren Salze, dadurch gekennzeichnet, daß man die Verbindungen der Formel II

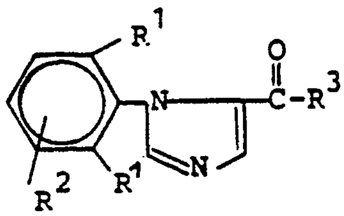

(II),

worin

$R^3$ = -O-$R^4$ -OH, Chlor,

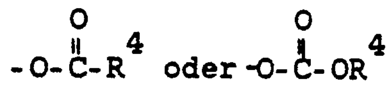

und

$R^4$ = $(C_1\text{-}C_8)$Alkyl bedeuten,

einer Ammonolyse unterwirft und die erhaltenen Verbindungen gewünschtenfalls in ihre Imidazolium-Salze überführt.

4. Pflanzenwachstumsregulierende Mittel mit einem wirksamen Gehalt an einer Verbindung der allgemeinen Formel (I) oder deren Salze von Anspruch 1 oder 2.

5. Verwendung von Verbindungen der allgemeinen Formel (I) oder deren Salze von Ansprüchen 1 oder 2 als Wachstumsregulatoren für Pflanzen.

6. Verfahren zur Wachstumregulierung von Pflanzen, dadurch gekennzeichnet, daß man auf die Pflanzen oder die Anbaufläche eine wirksame Menge einer Verbindung der allgemeinen Formel (I) Ansprüchen 1 oder 2 appliziert.

Patentansprüche für folgenden Vertragsstaat : ES

1. Verfahren zur Herstellung von Verbindungen der Formel I

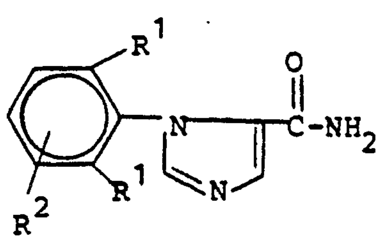

(I)

worin

$R^1$ = unabhängig voneinander $(C_1\text{-}C_3)$Alkyl,

$R^2$ = H, Methyl oder Halogen bedeuten

sowie deren für landwirtschaftliche Zwecke einsetzbaren Salze, dadurch gekennzeichnet, daß man die Verbindungen der Formel II

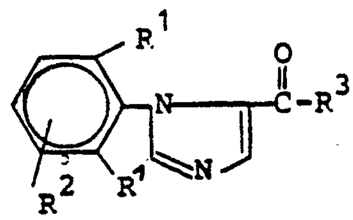

(II),

worin
$R^3$ = $-O-R^4$, -OH, Chlor,

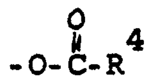 oder 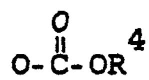

und
$R^4$ = $(C_1-C_8)$Alkyl bedeuten,
einer Ammonolyse unterwirft und die erhaltenen Verbindungen gewünschtenfalls in ihre Imidazolium-Salze überführt.

2. Verfahren gemäß Anspruch 1, wobei in Formeln I und II
$R^2$ = H bedeutet.

3. Verfahren zur Wachstumregulierung von Pflanzen, dadurch gekennzeichnet, daß man auf die Pflanzen oder die Anbaufläche eine wirksame Menge einer Verbindung der allgemeinen Formel (I) Ansprüchen 1 oder 2 appliziert.

Patentansprüche für folgenden Vertragsstaat : AT

1. Verfahren zur Herstellung von Verbindungen der Formel I

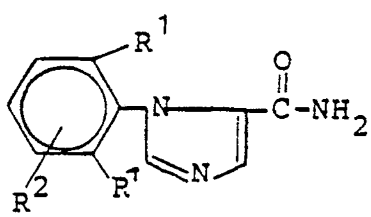

(I)

worin
$R^1$ = unabhängig voneinander $(C_1-C_3)$Alkyl,
$R^2$ = H, Methyl oder Halogen bedeuten
sowie deren für landwirtschaftliche Zwecke einsetzbaren Salze, dadurch gekennzeichnet, daß man die Verbindungen der Formel II

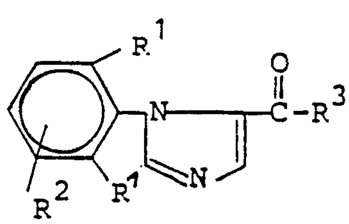

(II),

worin
$R^3$ = $-O-R^4$, -OH, Chlor,

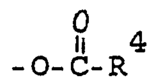 oder 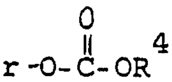

und
$R^4$ = ($C_1$-$C_8$)Alkyl bedeuten, einer Ammonolyse unterwirft und die erhaltenen Verbindungen gewünschtenfalls in ihre Imidazolium-Salze überführt.

2. Verfahren gemäß Anspruch 1, wobei in Formeln I und II $R^2$ = H bedeutet.

3. Pflanzenwachstumsregulierende Mittel mit einem wirksamen Gehalt an einer Verbindung der allgemeinen Formel (I) oder deren Salze von Anspruch 1 oder 2.

4. Verwendung von Verbindungen der allgemeinen Formel (I) oder deren Salze von Ansprüchen 1 oder 2 als Wachstumsregulatoren für Pflanzen.

5. Verfahren zur Wachstumregulierung von Pflanzen, dadurch gekennzeichnet, daß man auf die Pflanzen oder die Anbaufläche eine wirksame Menge einer Verbindung der allgemeinen Formel (I) von Ansprüchen 1 oder 2 appliziert.

## Claims

1. A compound of the formula I

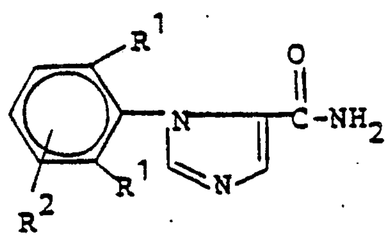

(I)

in which
the radicals $R^1$ independently of one another denote
($C_1$-$C_3$)alkyl and
$R^2$ denotes H, methyl or halogen, or a salt thereof which can he used for agricultural purposes.

2. A compound of the formula I or a salt thereof, in which $R^2$ denotes H.

3. A process for the preparation of a compound of the formula I or of a salt thereof, which comprises subjecting a compound of the formula II

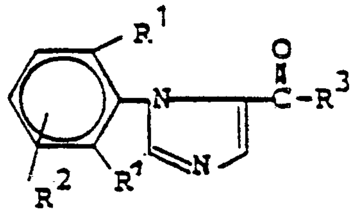

(II)

in which
$R^3$ denotes -O-$R^4$, -OH, chlorine,

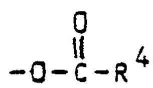 or 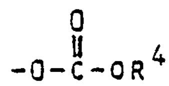

and
$R^4$ denotes ($C_1$-$C_8$)alkyl,
to ammonolysis and, if desired, converting the resulting compound into one of its imidazolium salts.

4. A plant growth-regulating agent with an effective content of a compound of the general formula (I) or a salt thereof as claimed in either of claims 1 and 2.

5. The use of a compound of the formula (I) or of a salt thereof as claimed in either of claims 1 and 2 as a growth regulator for plants.

6. A method for regulating the growth of plants, which comprises applying an effective amount of a compound of the formula (I) as claimed in either of claims 1 and 2 to the plants or the cultivation areas.

Claims for the following Contracting State : ES

1. A process for the preparation of a compound of the formula(I)

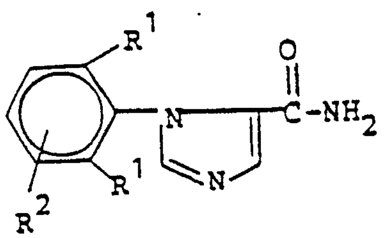

(I)

in which
the radicals $R^1$ independently of one another denote ($C_1$-$C_3$)alkyl and
$R^2$ denotes H, methyl or halogen or of a salt thereof, which comprises subjecting a compound of the formula II

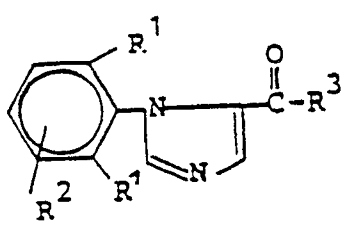

(II)

in which
$R^3$ denotes -O-$R^4$, -OH, chlorine,

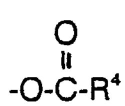 or 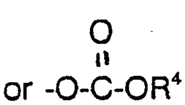

and
$R^4$ denotes ($C_1$-$C_8$)alkyl,
to ammonolysis and, if desired, converting the resulting compound into one of its imidazolium salts.

2. A process as claimed in claim 1, wherein in formulae (I) and (II) $R^2$ denotes H.

3. A process for regulating the growth of plants, which comprises applying an effective amount of a

compound of the formula (I) as claimed in either of claims 1 and 2 to the plants or the cultivation areas.

Claims for the following Contracting State : AT

**1.** A process for the preparation of a compound of the formula(I)

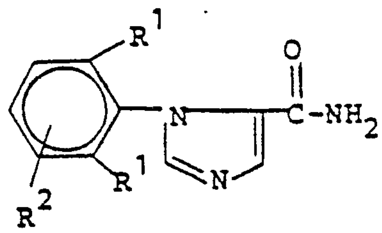

(I)

in which
the radicals $R^1$ independently of one another denote $(C_1\text{-}C_3)$alkyl and
$R^2$ denotes H, methyl or halogen, or of a salt thereof, which comprises subjecting a compound of the formula(II)

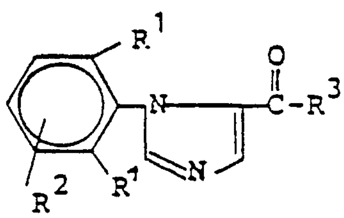

(II)

in which
$R^3$ denotes $-O-R^4$, -OH, chlorine,

$$-O-\overset{O}{\overset{\|}{C}}-R^4 \text{ or } -O-\overset{O}{\overset{\|}{C}}-OR^4$$

and
$R^4$ denotes $(C_1\text{-}C_8)$alkyl,
to ammonolysis and, if desired, converting the resulting compound into one of its imidazolium salts.

**2.** A process as claimed in claim 1, wherein in formulae (I) and (II) $R^2$ denotes H.

**3.** A plant growth-regulating agent with an effective content of a compound of the general formula (I) or a salt thereof as claimed in either of claims 1 and 2.

**4.** The use of a compound of the formula (I) or of a salt thereof as claimed in either of claims 1 and 2 as a growth regulator for plants.

**5.** A method for regulating the growth of plants, which comprises applying an effective amount of a compound of the formula (I) as claimed in either of claims 1 and 2 to the plants or the cultivation areas.

**Revendications**

**1.** Composés répondant à la formule I

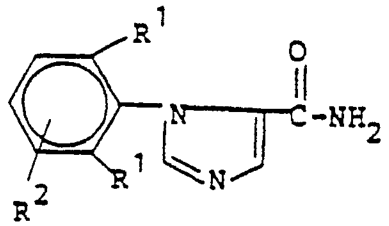 (I)

dans laquelle

les $R^1$ représentent chacun, indépendamment l'un de l'autre, un alkyle contenant de 1 à 3 atomes de carbone, et

$R^2$ représente H, un méthyle ou un halogène, ainsi que leurs sels utilisables pour des applications en agriculture.

2. Composés de formule I dans lesquels $R^2$ représente H, et sels de ces composés.

3. Procédé pour préparer des composés de formule I et leurs sels, procédé caractérisé en ce qu'on soumet à une ammoniolyse des composés répondant à la formule II :

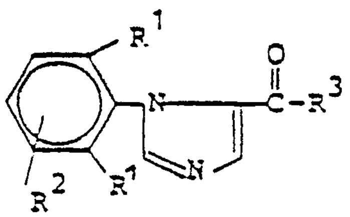 (II)

dans laquelle

$R^3$ représente un radical

$-O-R^4$, $-OH$, Cl, 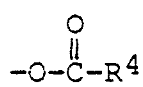

ou

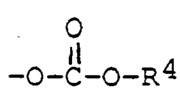

et

$R^4$ représente un alkyle contenant de 1 à 8 atomes de carbone,

et, si on le désire, on transforme les composés obtenus en leurs sels d'imidazoliums.

4. Produits régulateurs de là croissance des plantes, produits qui contiennent une quantité efficace d'un composé de formule générale I ou de sels de celui-ci selon l'une des revendications 1 et 2.

5. Application de composés de formule générale I et de leurs sels selon l'une des revendications 1 et 2 comme régulateurs de croissance pour plantes.

6. Procédé pour régler la croissance de plantes, procédé caractérisé en ce qu'on applique sur les plantes ou sur la surface de culture une quantité efficace d'un composé de formule générale I selon l'une des revendications 1 et 2.

Revendications pour l'Etat contractant suivant : ES

1. Procédé pour préparer des composés répondant à la formule I :

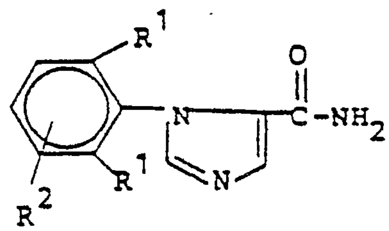

(I)

dans laquelle

les $R^1$ représentent chacun, indépendamment l'un de l'autre, un alkyle contenant de 1 à 3 atomes de carbone, et

$R^2$ représente H, un méthyle ou un halogène, ainsi que leurs sels utilisables pour des applications en agriculture, procédé caractérisé en ce qu'on soumet à une ammoniolyse des composés répondant a la formule II :

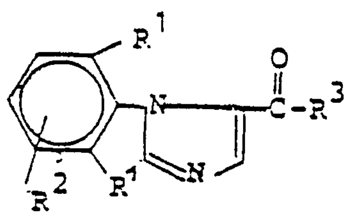

(II)

dans laquelle

$R^3$ représente un radical

$$-O-R^4,\quad -OH,\quad Cl,\quad -O-\overset{O}{C}-R^4$$

ou

$$-O-\overset{O}{\overset{\|}{C}}-O-R^4$$

et

$R^4$ représente un alkyle contenant de 1 à 8 atomes de carbone,

et, si on le désire, on transforme les composés obtenus en leurs sels d'imidazoliums.

2. Procédé selon la revendication 1 caractérisé en ce que, dans les formules I et II, $R^2$ représente l'hydrogène.

3. Procédé pour régler la croissance de plantes, procédé caractérisé en ce qu'on applique sur les plantes ou sur la surface de culture une quantité efficace d'un composé de formule générale I selon l'une des revendications 1 et 2.

Revendications pour l'Etat contractant suivant : AT

1. Procédé pour préparer des composés répondant à la formule I :

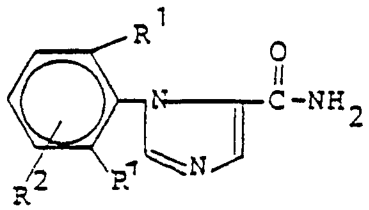

(I)

dans laquelle

les $R^1$ représentent chacun, indépendamment l'un de l'autre, un alkyle contenant de 1 à 3 atomes de carbone, et

$R^2$ représente H, un méthyle ou un halogène, ainsi que leurs sels utilisables pour des applications en agriculture, procédé caractérisé en ce qu'on soumet à une ammoniolyse des composés répondant à la formule II :

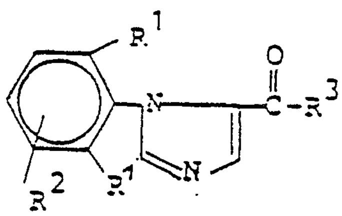

(II),

dans laquelle

$R^3$ représente un radical

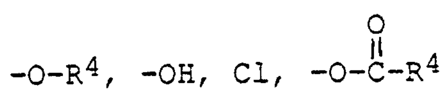

ou

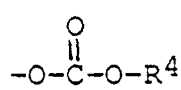

et

$R^4$ représente un alkyle contenant de 1 à 8 atomes de carbone,

et, si on le désire, on transforme les composés obtenus en leurs sels d'imidazoliums.

2. Procédé selon la revendication 1 caractérisé en ce que, dans les formules I et II, $R^2$ représente l'hydrogène.

3. Produits régulateurs de la croissance des plantes, produits qui contiennent une quantité efficace d'un composé de formule générale I ou de sels de celui-ci selon l'une des revendications 1 et 2.

4. Application de composés de formule générale I et de leurs sels selon l'une des revendications 1 et 2 comme régulateurs de croissance pour plantes.

5. Procédé pour régler la croissance de plantes, procédé caractérisé en ce qu'on applique sur les plantes ou sur la surface de culture une quantité efficace d'un composé de formule générale I selon l'une des revendications 1 et 2.